Europäisches Patentamt

European Patent Office

Office européen des brevets

(11) Publication number: **0 138 989 B1**

# EUROPEAN PATENT SPECIFICATION

(45) Date of publication of patent specification: **26.08.92**

(51) Int. Cl.⁵: **A61M 1/00**, B65B 31/04, B67C 3/16, F01L 7/08

(21) Application number: **84901673.8**

(22) Date of filing: **28.03.84**

(86) International application number:
**PCT/US84/00489**

(87) International publication number:
**WO 84/03838 (11.10.84 84/24)**

Divisional application 90102133.7 filed on
28/03/84.

(54) **DRAINAGE DEVICE FOR USE WITH SUCTION OR GRAVITY FLOW.**

(30) Priority: **28.03.83 US 479683**

(43) Date of publication of application:
**02.05.85 Bulletin 85/18**

(45) Publication of the grant of the patent:
**26.08.92 Bulletin 92/35**

(84) Designated Contracting States:
**AT BE CH DE FR GB LI LU NL SE**

(56) References cited:
| | |
|---|---|
| **EP-A- 0 065 386** | **GB-A- 1 363 890** |
| **GB-A- 2 082 071** | **US-A- 18 526** |
| **US-A- 603 391** | **US-A- 3 068 702** |
| **US-A- 4 289 158** | **US-A- 4 372 336** |
| **US-A- 4 396 386** | **US-A- 4 405 309** |

(73) Proprietor: **Pfizer Hospital Products Group, Inc.**
**235 East 42nd Street**
**New York New York 10017(US)**

(72) Inventor: **BLAKE, Larry, W.**
**10 Lexington**
**Irvine, CA 92714(US)**
Inventor: **HARVEL, Ervin, R.**
**23991 Lindley**
**Mission Viejo, CA 92675(US)**
Inventor: **MASON, Duane, R.**
**31 Farragut**
**Irvine, CA 92664(US)**
Inventor: **WRIGHT, George, M.**
**24145 Puerta Deluz**
**Mission Viejo, CA 92675(US)**

(74) Representative: **Rushton, Ronald et al**
**SOMMERVILLE & RUSHTON 11 Holywell Hill**
**St. Albans Hertfordshire AL1 1EZ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

This invention is concerned with a drainage apparatus for extracting fluids from the body, particularly from the pleural cavity in the human body.

### Background of the Invention

Artificial drainage of excess fluids from the body is necessary for the well being of the patient in a variety of medical procedures during and after operations and when the body cannot dispose of such fluids naturally. Fluids to be drained include blood, water and air.

A wide variety of drainage devices have been developed to drain wounds and larger body cavities. These devices comprise, in general, a chamber for collection of the fluids and a inlet tube for connection between the patient and the chamber. In use, the fluids are drained from the patient via the inlet tube into the collection chamber which is placed well below the patient to avoid syphoning the fluids back to the patient. Drainage may be caused by gravity, since the chamber is below the patient or, more efficiently, by applying suction to an outlet from the collection chamber to draw the fluid into the chamber.

Various thoracic or cardiac surgical procedures as well as trauma wounds, such as bullet or stab wounds accumulate fluids, such as blood, water or air, in the pleural or thoracic cavities. In the pleural cavity, between the lungs and surrounding chest wall, this accumulation can interfere with breathing and can cause death by inhibiting the normal pressure changes in the pleural cavity which support normal lung function. In the thoracic cavity, accumulation of fluid such as blood can inhibit healing and patient recovery.

In the case of a persistent leakage of air into the pleural cavity, such as from a ruptured bleb - a blister on the lung - the pressure builds up in the cavity until a tension pneumothorax is caused. Then the affected lung, or even both lungs, collapse and the patient can suffocate unless the air is drained from the pleural cavity.

The design of a drainage apparatus for the pleural or thoracic cavities present special problems. To be effective, the drainage apparatus must remove the fluids promptly and efficiently and prevent their return. It is essential to have one way flow not only to isolate the patient from the atmosphere, so as to avoid infection, but also to prevent fluid, particularly air, from collecting in the pleural cavity and allowing the lungs to collapse.

The simplest form of such a drainage apparatus comprises a single bottle partially filled with water and serving as a water seal chamber. The bottle has an inlet tube from the patient extending below the water level and an outlet tube communicating with the air space above the water. This is a rudimentary arrangement which provides no control over, or indication of, the pressure in the collection chamber. As the drained material gradually fills the bottle, the resistance to drainage increases. To improve on this design, a plurality of bottles have been used, connected in series between the patient and a source of suction. Normally three bottles are used: the first, connected to the patient as a trap, collects the fluids; the second, partially filled with water, forms the seal; and the third acts as a pressure regulator by having a tube open to the atmosphere and extending below water in the bottle. The depth of this tube below the water determines the maximum amount of suction that can be drawn through the system because once sufficient suction is reached to drain air down the tube from the atmosphere and bubble through the water, increasing the level of suction only increases the rate of bubbling and consequently the level of suction in the apparatus is limited. However, the separate bottle arrangement is cumbersome and awkward to use and is very difficult to clean for reuse. This has led to the design of a variety of unitary, sterilizeable and disposable drainage apparatus.

U.S. Patent No. 2,936,757 discloses such an apparatus in the form of a disposable, flexible plastic bag containing inner pockets for retaining the water seal and having access tubes reaching into those pockets. Unfortunately, the bag contains a vent to the atmosphere, which can lead to contamination of the patient. A flexible material is unsuitable for a water seal drain since jarring the device can displace the water and interrupt use of the drain.

U.S. Patents No. 3,363,626, 3,363,627, 3,559,647, 3,683,913 and 3,853,128 show the development of a rigid, disposable, plastic drainage apparatus which has adapted the three-bottle arrangement into a unitary structure. Each apparatus in these patents comprises a fluid collection chamber, a U-shaped water seal compartment and a U-shaped water manometer compartment for suction control. The fluid collection chamber is typically divided into three, graduated compartments which, in use, fill sequentially as the amount of fluid drained accumulates.

A drainage apparatus which includes a water seal, requires a relatively long time to set up because the apparatus must be primed with a certain volume of sterile water to generate the seal and to fill the U-shaped chamber. This volume of water is relatively large and usually more than one chamber has to be filled. Also, the amount of suction that can be applied to a water seal system is limited by the depth of water in the pressure regulator chamber, as mentioned above. The maximum suction that can be applied to commercial water seal reservoirs is about 30 cm. water, since a much longer pressure control chamber is very cumbersome and expensive to manufacture. This is inadequate since suction up to about 60 cm. water is sometimes required.

Further, a water seal drainage apparatus is normally relatively heavy and large which is a disadvantage both for hospital staff and for the patient who often holds the apparatus when he is moved. Another serious drawback is the relatively complex instructions required for its use which can lead to mistakes and wasted time.

In attempts to overcome these deficiencies, a few waterless devices have been developed. U.S. Patent No. 3,830,328 discloses a simple arrangement which is a waterless, one-chamber device containing an inlet tube having a one-way valve to prevent flow of fluid back to the patient and a bellows arrangement to coarsely measure the pressure in the pleural cavity. But this device does not control or accommodate pressure fluctuations in the chamber.

A recent device without a water seal has been produced by Davol, Inc. under the trademark Thoraklex and comprises a chamber for collecting drained fluids which are supplied to the chamber through an inlet having a U-tube inside the chamber for holding a small amount of water to indicate an air leak in the system, and an outlet to suction through a one-way valve. A rubber plug in the wall of the U-tube provides access for a syringe needle to extract liquid from the tube for analysis. The outlet passage is connected through a one-way vent to the atmosphere to provide a positive pressure relief valve. The outlet is also connected to a negative pressure control consisting of a screw for adjustably occluding the outlet passage to regulate the degree of suction. The outlet also includes a float ball gauge calibrated to indicate the suction applied to the patient and connected between the atmosphere and the outlet. This apparatus incorporates an over-negative-pressure relief valve which vents to atmosphere at a pre-set negative pressure to prevent the vacuum reaching too high a level. This valve is set in a passage between the atmosphere and the collection chamber and comprises a one-way spring loaded valve. Since actuation of the valve allows air from the atmosphere into the collection chamber which is open to the patient, it is necessary to provide a filter in the passage. This arrangement suffers from the disadvantages that the suction level can build up rapidly in the collection chamber while the valve is opening and although the filter is intended to prevent bacteria entering the chamber, placement of the valve opening directly to the chamber still exposes the patient to risk of infection and also increases manufacturing costs of the apparatus. A device of this construction is also disclosed in U.K. Patent Application GB 2,082,071 A.

There is therefore a need for a drainage apparatus which avoids the disadvantages of the water seal apparatus and yet which functions reliably and safely. The apparatus must be able when functioning under vacuum drainage to apply stable and acceptable levels of suction to the patient. In its broadest aspect the present invention aims to provide such a drainage apparatus and which overcomes the aforesaid disadvantages of the prior art.

According to the invention there is provided an apparatus for draining fluid from a cavity in the body of a patient, as defined in Claim 1.

The means for venting the outlet may comprise a seal over an opening in said outlet to the atmosphere, a spring biasing said seal to close said opening, and a screw which is adjustable to vary the compression of said spring and thereby the biasing force of said spring on said seal and consequently to set the degree of suction required to overcome said force to open said seal. The force of the spring against the seal may be adjustable by means of a screw bearing upon the end of the spring opposite the seal. A particular level of suction will be sufficient to overcome the force of the spring and the seal will then open to admit the atmosphere to the outlet and thereby relieve the suction.

To avoid contamination the means for allowing air to be drawn through the outlet from said chamber may comprise a one-way valve which allows air to pass out of the chamber but not into it.

The apparatus of this invention may have a gauge for indicating directly the pressure in the collection chamber and displaying the true level of suction applied to the patient, irrespective of whether an air leak from the patient is passing through the equipment.

The gauge may comprise a diaphragm which is flexible but is impervious to drained liquids and to air and which is mounted in the chamber wall for communication with both the inside of the chamber and the atmosphere. The diaphragm is connected to means for indicating the pressure. Pressure differentials between the atmosphere and the pressure inside the chamber cause the diaphragm to flex, increasing negative pressure in the chamber causing the diaphragm to bulge in towards the inside of the chamber, increasing positive or decreasing negative pressure causing it to bulge in the opposite direction. This movement of the diaphragm is translated to the indicating means to directly display the working pressure in the chamber which is usually less than atmospheric since the apparatus is primarily intended for connection to a source of suction. Preferably the indication is achieved by a worm gear rotatably mounted on the diaphragm to project upwardly and engage a follower which in turn is coupled to a pointer for indicating the pressure or degree of suction on a dial.

In embodiments of the invention the collection chamber may be provided with features which make placement, collection and transportation of the device more convenient. Thus the collection chamber may be provided with means which serve both to suspend the collection chamber and to carry it and yet which may also be stored flush against the chamber when not in use. Integral means may also be provided to stabilize the chamber against upset when standing on a support surface, such as the floor, without resort to time-consuming manipulation of the device or use of extraneous devices. Thus, suspension means may be rotatably mounted on the external surface of the chamber in such a manner that they will not only suspend the chamber but also can be nested on the external surface for storage and interlocked to form a carrying handle. To stabilize the chamber when placed on a support surface such as the floor or a trolley, a stand may be provided on the base of the chamber which is rotatable from a storage position in a recess in the base to project outwards and thereby prevent the chamber from being knocked over or tilted.

The invention will now be described by way of example with reference to the accompanying drawings, in which:

Figure 1 is a perspective view of a drainage apparatus illustrative of the invention and showing the apparatus for connection to a patient and a source of suction and showing means for suspending the device or stabilizing it on a support surface;

Figure 2 is a top view of the apparatus in Figure 1 with the connections and suspension means removed;

Figure 3 is a sectional view taken along the line 3-3 in Figure 2 showing an inlet to the apparatus;

Figure 4 is a fragmentary sectional view of the front elevation of an alternative form of inlet for the apparatus of Figure 1;

Figure 5 is a fragmentary sectional view of the front elevation of another form of inlet for the apparatus of Figure 1;

Figure 6 is a fragmentary sectional view taken along the line 6-6 in Figure 5;

Figure 7 is a fragmentary sectional view of the front elevation of a further alternative form of inlet for the apparatus of Figure 1;

Figure 8 is fragmentary a sectional view taken along the Line 8-8 in Figure 7;

Figure 9 is a sectional view taken along the line 9-9 in Figure 2;

Figure 10 is a sectional view taken along the line 10-10 in Figure 2 showing an outlet to the apparatus of Figure 1;

Figure 11 is a perspective view of an alternative outlet to the apparatus of Figure 1;

Figure 12 is a fragmentary sectional view of another form of outlet for the apparatus in Figure 1.

Detailed Description

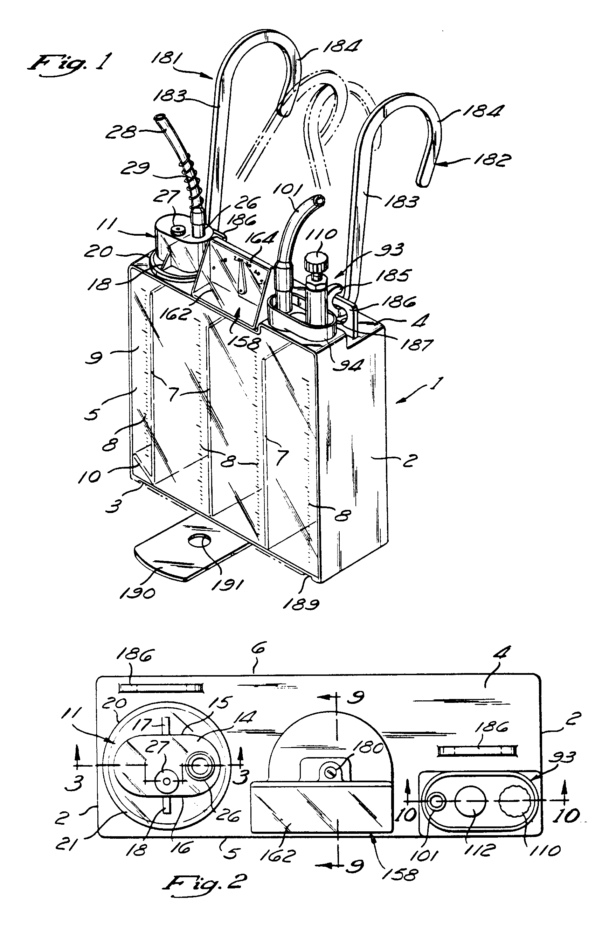

In Figure 1 there is shown a drainage apparatus suitable for draining fluids from the pleural cavity and comprising a fluid collection chamber 1 formed by side walls 2, a base 3, a top 4 and front and rear walls 5 and 6 (Figure 2), respectively. The chamber is preferably constructed from lightweight yet rigid plastic such as polystyrene so that the device can be relatively cheaply constructed by known molding techniques. The front wall 5 is transparent for viewing the contents of the chamber. The other walls, 2, 5, and 6, the base 3 and top 4 may be transparent or opaque. Preferably all sides of the chamber except the front walls are opaque so as to shield the contents from the patient's view and to provide a background against which the contents are more easily visible. The chamber is internally divided by partitions 7 between the front and rear walls 5 and 6 into compartments communicating with each other only over the top of each partition 7. In use, the compartments are sequentially filled by the accumulation of drained liquid. In Figure 1, three partitions 7 are shown joining front and rear walls 5 and 6 and dividing the chamber into four compartments, but fewer or more partitions 7 may be used. Graduations, as at 8, are provided up the front wall 5 on each compartment to indicate the volume of liquid collected. The chamber 1 shown in the figure preferably has graduations 8 marked up to a total of 2,000 ml, although, of course, smaller or larger chambers can be used. Alongside the graduations, space is provided on the front wall 5 for noting the time a particular volume is reached. Preferably, as shown in Figure 1, the first compartment 9 is smaller than the other compartments which are the same size as one another. This allows for more accurate and convenient collection of smaller or initial quantities of liquid, as may be the case in pediatric procedures. For more accurate collection of very small quantities of liquid, the floor of the first compartment may be angled upwards as at 10 to reduce the volume at the bottom of the compartment. Commensurate with this difference in volume between the compartments, the graduations on the first compartment 9 represent smaller volumes than those on the other compartments. For example, the graduations may run each 2 ml up to 10 ml, then every 5 ml up to 250 ml on the first compartment and then every 10 ml up to 2,200 ml on the other compartments.

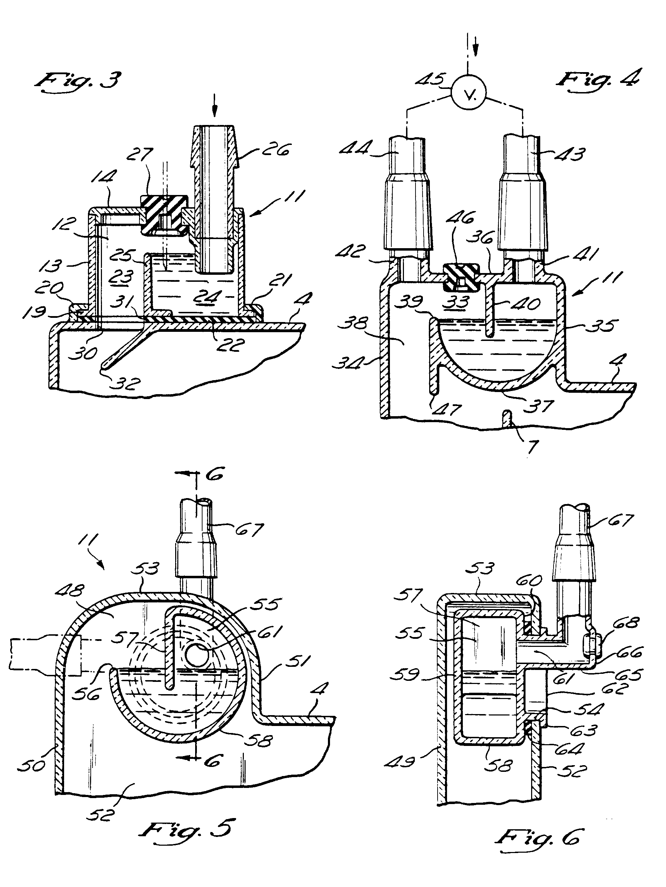

Above the first compartment 9 is a switchable inlet 11 on the top 4 of collection chamber 1 which allows the option of draining fluid through a small reservoir of liquid in the inlet or avoiding that small quantity of liquid. One such inlet 11 is shown in Figures 1, 2 and 3. The form of inlet shown in these Figures is preferred for its efficiency and ease of use. In this embodiment, inlet 11 is rotatable on the top 4 of collection chamber 1 in a plane parallel to that of top surface 4 and its construction can best be seen in Figure 3. There, inlet 11 comprises an inlet chamber 12 formed by an outer encircling perimeter wall 13, projecting upwardly from the top 4 of collection chamber 1, and a substantially flat top 14 across wall 13. Viewed from above top 4, as in Figure 2, wall 13, and consequently chamber 12 within, has a flattened, circular cross-section with two opposite and parallel sides 15 and 16 to facilitate the grasping of inlet 11 for rotation. For this purpose also, diametrically opposed projections 17 and 18 may be provided, as shown in Figure 2, which extend outwardly from the center of sides 15 and 16, respectively to be engaged by the hand of the user. However, if desired, the cross-section of wall 13 can be a regular circle or oval. The bottom of wall 13 has an outwardly projecting, circular flange 19 (Figure 3) around its periphery. Flange 19 is surrounded by a circular retaining ring 20 attached to top 4 of collection chamber 1. Ring 20 also has a flange 21, which projects inwardly and over flange 19 to retain the inlet chamber 12 on the top 4 of the collection chamber 1 and to also allow it to rotate thereon within retaining ring 20 and its flange 21. To prevent leakage of fluid from between inlet chamber 12 and collection chamber 1 to the outside of the chambers or nonsterile air from contaminating the chamber, a seal 22 is placed between inlet chamber flange 19 and top surface 4. The inlet chamber is divided into two compartments 23 and 24 by a dividing wall 25 across the diameter of chamber 12 and between sides 15 and 16. Dividing wall 25 extends upwardly from the bottom of the chamber but does not reach the top 14 of the chamber 12, thereby leaving above wall 25 an air space which is the only means of fluid communication between the two compartments 23 and 24 since the bottom of dividing wall 25 is sealed against seal 22. Above one compartment 24, an inlet connector 26 and a rubber grommet or sampling plug 27 extend through the top 14 of the inlet chamber 12. Connector 26 comprises a short rigid tube whose upper end is for attachment to an inlet tube 28 as shown in Figure 1. Inlet tube 28 is for connection to the patient and is made of flexible natural rubber or flexible polyvinylchloride to prevent the tube 28 from kinking at its junction with connector 26 and obstructing the flow of fluid from the patient, an internal or, as shown in Figure 1, external coil of resilient material such as plastic or metal wire 29 can be wrapped around inlet tube 28 to give the tube some rigidity at connector 26. The lower end of connector 26 extends below the top of dividing wall 25 in compartment 24. As liquid is drained from the patient down inlet tube 28, it is delivered into compartment 24. When that compartment fills and the level of liquid reaches the height of dividing wall 25, the liquid spills over wall 25 into the other compartment 23. Since connector 26 extends below the top of dividing wall 25, it also extends below the level of liquid in compartment 24 and forms with dividing wall 25 and inlet chamber wall 13, an inlet passage having a generally U-shaped cross-section, as shown in Figure 3. This U-tube arrangement serves as an indicator of the patient's condition since whenever liquid is present in the U-shaped inlet passage, a flow of bubbles in the direction from connector 26 through compartment 24 indicates an air leak from the patient. Moreover, motion of the liquid reflects patency and pressure changes in the patient's chest and can indicate if he is having breathing difficulties. The U-shaped passage is preferably sized to contain 10-15 ml of liquid and may be graduated to indicate the volume of liquid in it, for example in divisions of 2 mls up to 10 mls. Instead of having the connector 26 extend so far into compartment 24, in an alternative embodiment (not shown), the U-tube can be formed by a sub-dividing wall projecting down from the top of inlet chamber 12 below the top of dividing wall 25 and between that wall and outer wall 13 with inlet connector 26 being between the outer wall and the sub-dividing wall. Grommet 27 is made of a rubbery material, such as natural or silicone rubber, which allows a hypodermic syringe needle (shown in phantom in Figure 3) to pass through it and be withdrawn without exposing inlet chamber 12 to the atmosphere. By means of the syringe, most recently drained liquid can be sampled anytime by withdrawing some liquid from compartment 24. When no liquid is drained and to prime the apparatus for use, sterile saline solution can be injected into compartment 24 through grommet 27 in an amount sufficient to fill the bottom of compartment 24 and so that an air leak from the patient can be indicated by bubbling through the liquid, as described above.

Inlet 11 is located on top 4 of collection chamber 1 so that one of compartments 23 and 24 is above a hole 30 through top 4 and a hole 31 through seal 22. Neither of these compartments has a floor so that the bottom of one of them will be open through holes 30 and 31 while the bottom of the other compartment is closed by seal 22 and the top 4 of collection chamber 1. When compartment 24 fills during drainage as described above and the drained liquid spills over the dividing wall to compartment 23, the liquid can then fall through holes 30 and 31 into collection chamber 1. Hole 30 is positioned so that it is above or nearly above the first collection compartment, so that this compartment fills first. To ensure this, a dip 32, shown in Figure 3, is angled downwards below hole 30 from the underside of top 4 to direct the liquid into the first collection compartment 9 (Figure 1).

A great advantage of the apparatus is that the inlet 11 can hold liquid for sampling or to indicate an air leak from the patient and that liquid can be removed, such as by being emptied from the inlet into the collection chamber, when no longer needed or to dispose of a potential blockage of the inlet caused by matter such as clots or other debris in the drained liquid. Since inlet 11 in Figure 3 is rotatable, when it is turned through 180°, the position of the compartments 23 and 24 will be reversed. Thus, compartment 24 will be brought over hole 30 and the liquid in it will be dumped into collection chamber 1. In this position, the bottom of the other compartment 23 is closed by seal 22 and fluid from the patient passes straight through compartment 24 into collection chamber 1, i.e., without being temporarily retained in an inlet passage or passed through liquid there, but passing straight into the main collection chamber from the inlet tube from the patient. When it is desired to revert to the sampling or air leak display mode or after clearing the inlet of an obstruction, inlet 11 is turned again through 180°, thus reversing the positions of compartments 23 and 24 and returning them to the positions shown in Figure 3. Then, drained fluid first enters compartment 24 and may be allowed to refill that compartment, or fresh sterile saline solution may be injected into compartment 24 to fill the "U-tube" inlet passage. Inlet 11 is constructed so that the inlet cannot be blocked off at any time through rotation of the inlet.

An alternative form of switchable inlet is shown in Figure 4, in an arrangement where the liquid in the inlet passage can be by-passed by the fluid drained from the patient. An inlet chamber 33 is defined by the following: a clear front wall (not shown), which is an upward extension of part of the clear front wall 5 of collection chamber 1; an outer side wall 34, which is a similar extension of the collection chamber's side wall 2 and, extending upwardly from top 4 of chamber 1, an inner side wall 35 and a rear wall (not shown) and a substantially flat top 36. Side walls 34 and 35 extend somewhat less than half the depth of top 4 of collection chamber 1. An inlet passage of generally U-shaped cross-section, when viewed from the front of the apparatus as in Figure 4, is formed in inlet chamber 33 by a curved trough 37 which extends between front and rear walls of the chamber 33, and partway across the chamber from inner side wall 35, leaving a space 38 between the lip 39 of trough 37 and outer side wall 34. A dividing wall 40 projects downwardly from the underside of top 36 of inlet chamber 33 part way into trough 37 and below its lip 39 and also extends between the front and rear walls of chamber 33. Thus, in vertical cross-section, trough 37, dividing wall 40 and inner side wall 35, form a generally U-shaped inlet passage. As shown in Figure 4, two inlets 41 and 42 pass through the top 36 of inlet chamber 33: a primary inlet 41 over the inlet passage and trough 37 and between dividing wall 40 and inner side wall 35 and a secondary inlet 42 over the space 38 between trough 37 and outer side wall 34. Attached to inlets 41 and 42 are lengths of flexible tubing 43 and 44, respectively, which lead to a common joint in the form of a switching valve 45 to which is also attached the main drainage tube from the patient. Valve 45 can be used to direct drained fluid either through primary inlet 41 and the U-tube inlet passage or through secondary inlet 42 and space 38 directly into collection chamber 1. In using the primary inlet 41, drained fluid passes through liquid in trough 37, previously drained or placed there. This liquid seal can act as an air leak indicator or a point for analysis of drained liquid, as described above. Liquid can be withdrawn from or placed in trough 37 through a rubber grommet 46 in the top 36 of inlet chamber 33. Grommet 46 is located between dividing wall 40 and secondary inlet 42 and above the outlet arm of the U-tube inlet passage between dividing wall 40 and lip 39 of trough 37. Drained fluid passes through the inlet passage, space 38, and into the first collection compartment 9 below. A downward projection 47 from the lip 39 of trough 37 above compartment 9 assists in directing drained liquid into this compartment. If use of the inlet passage is not desired because it is obstructed or the air leak or sampling facility is not needed, secondary inlet 42 can be used by turning valve 45 to direct the flow of drained fluid to that inlet rather than the primary inlet 41. Then, the fluid passes directly through space 38 into collection compartment 9 without passing through liquid in the inlet passage.

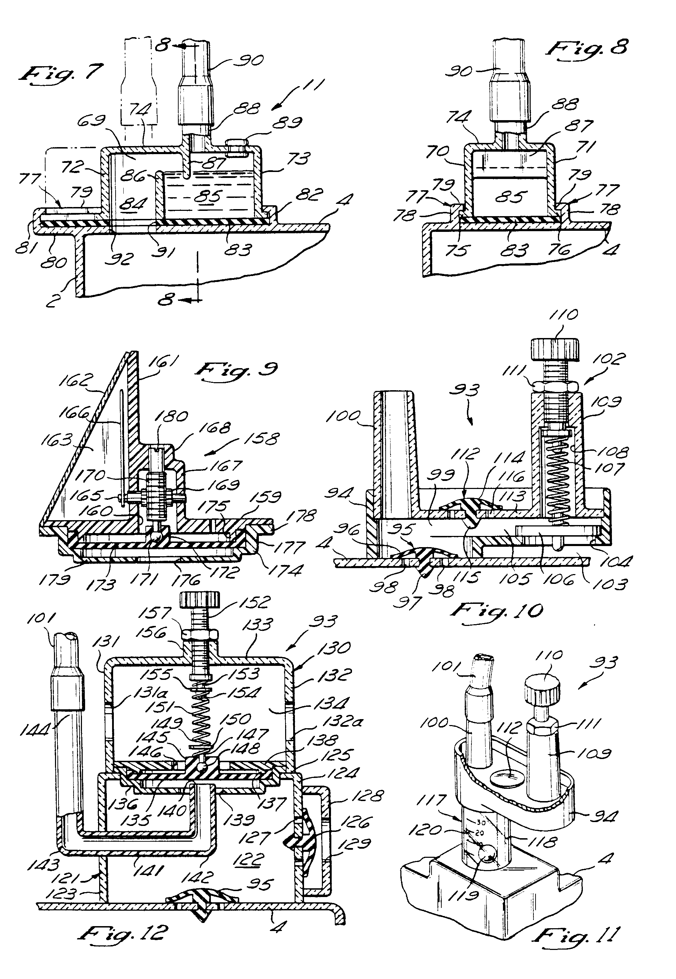

Another inlet is shown in Figures 5 and 6 in which the U-tube inlet passage is rotatable in a plane parallel to that of the front wall 5 of collection chamber 1 so that the inlet passage can be emptied. In a similar manner to that of the previously described inlet, the inlet chamber 48 is formed by a clear front wall 49 (Figure 6), which is an upward extension of the front wall 5 (Figure 1) of collection chamber 1, an outer side wall 50 extending upwardly from the side wall 2 (Figure 1) of the collection chamber 1 and an inner side wall 51 and a rear wall 52, each extending upwardly from the top 4 of chamber 1. As shown in Figure 5, the top 53 of inlet chamber 48 is curved outwardly with respect to the chamber but a flat top could be used, provided there was sufficient clearance for rotation of the U-tube inlet passage, as will become apparent. Side walls 50 and 51 extend somewhat less than half the depth of top 4 of collection chamber 1. Rear wall 52 has a circular hole 54 through which is rotatably mounted an inlet compartment 55. Viewed from the front, as in Figure 5, compartment 55 has a vertical cross-section in the form of three-quarters of the circumfrence of a circle. As used in the position shown in Figure 5, compartment 55 terminates at the nine o'clock position in a lip 56 over which drained liquid spills into the first collection compartment 9 (Figure 1) below. At the twelve o'clock position, compartment 55 has a downwardly projecting dividing wall 57 extending below the level of lip 56. Between these two clock positions a continuous, curved wall 58 defines the outside of inlet compartment 55, together with front and rear walls 59 and 60, respectively. Inlet compartment 55 is thereby closed except at its inlet 61, through rear wall 60 between dividing wall 57 and outer wall 58, and its outlet between wall 57 and lip 56. Consequently, compartment 55 defines an inlet passage again having a generally U-shaped vertical cross-section, as viewed in Figure 5, so that an air leak from the patient will be indicated in the inlet compartment 55 by bubbling through the liquid therein as described above. So that the contents of the inlet passage can be seen, at least front walls 49 and 59 of the inlet chamber and compartment, respectively, are clear. If desired, all the walls of the inlet chamber and compartment can be clear, although it is preferred that the rear wall 52 of the inlet chamber be opaque to shield the contents from the patient's view. As can be seen in Figure 6, compartment 55 has a circular projection 62 passing through rear wall 52 of inlet chamber 48. Projection 62 is sized to fit snugly in hole 54 in rear wall 52 and terminate outside inlet chamber 48 in a flange 63 which overlaps and engages the outer surface of rear wall 52. Since hole 54 is smaller than flange 63 and the rear wall 60 of compartment 55, the compartment can rotate within hole 54 and be retained there. To facilitate this and to seal hole 54 to the atmosphere, an annular washer 64 of a suitably resilient material is placed between rear wall 60 of compartment 55 and the rear wall 52 of the inlet chamber 48. Inlet 61 to compartment 55 passes through projection 62 as a tubular conduit 65 before turning through a right angled elbow 66 to continue upwardly for accepting a flexible inlet tube 67, from the patient. A rubber grommet 68 or sampling plug is provided through the wall of elbow 66 facing the inlet 61 into the compartment 55 so that a syringe needle can supply liquid to or withdraw liquid from compartment 55, as described above. In use, liquid drained from the patient passes down inlet tube 67, through conduit 65 into compartment 55 and through the U-tube inlet passage before spilling over the lip 56 and into the collection compartments below. If it is desired to remove an obstruction from the compartment 55 or replace the liquid therein, compartment 55 can be rotated in a counterclockwise direction viewed from the front of the apparatus to a position shown in phantom in Figure 5, where compartment 55 is rotated through about 90° to spill its contents into the collection chamber 1. Then if it is desired to minimize obstruction to passage of drained fluid through the inlet, compartment 55 may be held in this tipped position without liquid therein allowing the drained fluid to flow directly into the collection chamber 1.

A further inlet is shown in Figures 7 and 8 which is similar in design to that of Figure 3, except that the inlet slides in a plane parallel to that of the top 4 of collection chamber 1, instead of rotating, to empty the inlet chamber. Inlet chamber 69 is formed by front and rear walls 70 and 71, respectively (Figure 8), and side walls 72 and 73 (Figure 7) with a substantially flat top 74 across these walls. Preferably, at least front wall 70 is clear to view the contents of inlet chamber 69 and the rear wall 71 is opaque to shield the contents from the patient's view and to provide a background rendering the contents more visible. Side walls 72 and 73 may be clear or opaque. As shown in Figure 8, front and rear walls 70 and 71 each have at their base outwardly projecting flanges 75 and 76 respectively. Flanges 75 and 76 are straight and parallel and lie within parallel guide rails 77, attached to the top 4 of collection chamber 1. Each guide rail comprises an upright projection 78 with a flange 79 extending inwardly and over inlet chamber flanges 75 and 76. Inlet chamber 69 is thus held on collection chamber 1 but is free to slide between the guide rails 77. The top 4 of collection chamber 1 has an extension 80 which overhangs the side wall 2 of chamber 1. Guide rails 77 continue from the main top surface 4 onto overhang 80. The extent of travel of inlet chamber 69 along guide rails 77 is determined by stops 81 and 82 at each end of rails, one 81 on overhang 80 and the other 82 on top 4 over the collection compartments. In the preferred form shown in Figures 7 and 8, flanges 75 and 76 join flanges on the side walls of inlet chamber 69 to form a continuous flange around the bottom of chambers 69's walls. Similarly, stops 81 and 82 have inwardly projecting flanges which form together with flanges 79 on guide rails 77, a continuous flange which can overlay the continuous flange around chamber 69. To prevent leakage of fluid from between the inlet chamber 69 and top 4 of collection chamber 1, a seal 83 is placed between the inlet chamber flanges 75 and 76 and the top surface 4. Inlet chamber 69 is divided into two internal compartments 84 and 85, as shown in Figure 7, by a dividing wall 86 across the chamber 69 between and substantially perpendicular to front and rear walls 74 and 75. Dividing wall 86 extends upwardly from the bottom of the chamber 69 but does not reach the top 74 of the chamber, thereby leaving above wall 86 an air space which is the only means of fluid communication between the two compartments 84 and 85. The innermost compartment 85, with respect to top 4 of collection chamber 1, is divided by a sub-dividing wall 87 into an inlet passage having a generally U-shaped vertical cross-section when viewed from the front as in Figure 7, because sub-dividing wall 87 projects downwardly from top 74 of the inlet chamber 69 and extends part way down compartment 85 to below the top of dividing wall 86. Between sub-dividing wall 87 and the inner side wall 73 of the inlet chamber 69, an inlet 88 and rubber grommet 89 extend through the top 74 of chamber 69. Inlet 88 is for attachment of an inlet tube 90 from the patient and grommet 89 is used to withdraw liquid from or supply liquid to the inlet passage described above with respect to the other inlets. When the inlet chamber 69 is against the innermost guide rail stop 82, compartment 84 is above holes 91 and 92 and through seal 83 and top 4 of collection chamber 1, respectively. In this position, since neither of compartments 84 and 85 have a floor, drained fluid passes from inlet 88 through the inlet passage in compartment 85 and then through compartment 84 and hole 92 into the collection chamber 1. Drained liquid accumulates in compartment 85 whose bottom is closed by seal 83, and when the level reaches the top of dividing wall 86, it spills into the other compartment 84 through hole 92 and into the first collection compartment 9 (Figure 1) under hole 92. When it is desired to empty compartment 85 or to have drained liquid pass straight through inlet chamber 69 without accumulation there, the inlet chamber 69 is slid between guide rails 77 until it contacts the outer stop 81. In this position, compartment 84 is moved onto overhang 80 and its bottom closed by the seal 83 on overhang 80, while compartment 85 is moved over hole 92 and the contents of that compartment are emptied through that hole into collection chamber 1. Thus, any obstruction in the inlet passage may be cleared in this manner and, as above, this inlet provides the option of periodic or continuous collection of drained liquid for sampling or indication of air leaks from the patient in a body of liquid in the inlet in one mode, or the periodic or continuous passage of drained fluid through the inlet without use of such a body of liquid.

The apparatus is designed so that suction can be used to drain the fluids and for this purpose the apparatus shown in Figure 1 has an outlet 93 for connection to a source of suction (not shown). Nevertheless, the apparatus is constructed so that it is able to operate under gravity drainage in case the source of suction fails. The outlet, 93 comprises a raised housing 94 located on the top 4, and towards the front 5 of the collection chamber over the last compartment therein. As can be seen more clearly in Figure 10, the housing 94 places the outlet 93 above a one-way seal valve 95 in the top of the collection chamber. The valve 95 comprises a resilient cap 96 outside the chamber 1 and having an integral plug passing through the top of the chamber 1 and terminating inside the chamber 1 in an enlarged head 97 to retain the valve 95. The cap 96 covers perforations 98 in the top of the chamber 1 and seals them against back flow of fluid in the direction from the outlet 93 to the chamber 1. Valve 95 is located in an internal cavity 99 in outlet housing 94. Cavity 99 above valve 95 is in fluid communication into an outlet connector 100 which is for connection to a flexible outlet tube 101 (Figure 1) which in turn is for connection to the source of suction. Upon application of suction to the outlet 93 the edges of the cap 96 are drawn upwardly to expose the perforations 98 and, since they and outlet 93 are in communication through internal cavity 99, suction can be applied to the interior of the collection chamber 1. The valve 95 provides a slight interference between the resilient cap 96 and the top of the reservoir housing which results in a positive1 to 2 cm of positive pressure from patient commonly known as "cracking pressure" when the apparatus is used for gravity drainage. This is the same as the water mechanics.

The amount of suction applied to the collection chamber 1 is controllable by an adjustable regulator 102, best shown in Figure 10, located in the outlet housing 94. As shown in that figure, the portion of the housing 94 containing the regulator 102 is raised above the top of the chamber 1 to form a gap 103 therebetween. The floor of the housing 94 above this gap has a hole 104. Hole 104 opens to an internal channel 105 in the housing 94 is open to the cavity 99 and thus to the outlet connector 100 and the source of suction. The hole 104 in the floor of the housing 94 communicates between the gap 103 and the channel 105, and is closable by a seal valve 106 biased by a coil spring accommodated in a passage 108 within a column 109 extending upwards from the channel 105 and above the valve 106. The passage 108 accepts an adjustment screw 110 in contact with the end of the spring 109 remote from the valve 106. Screw 110 is adjustable through a nut 111 fixed to the outside and top of column 109. Adjustment of the screw 110 increases or decreases the pressure of the spring 107 against the valve 106. Thus a certain level of vacuum applied to the outlet 93 will overcome this pressure and cause the seal 106 to open, thereby venting the outlet 93 to atmosphere through the gap 103 to relieve the suction to a lower level at which the pressure of the spring 107 overcomes the suction and closes the seal 106. Consequently the regulator 102 may be set to open the valve 106 at a predetermined vacuum level to ensure that the suction applied to the chamber is not too great. This is a useful feature since many hospitals pipe suction around the building to be available at certain points in the walls without adjustment capability or regulations at these terminal points may only be adjustable from about 80 to 200 in water suction and not accurate at the desired levels of suction appropriate for chest drainage. Since this available suction, known as "wall suction", is often undesirably high, for example 200 inches (500 cms) of water or more when 10 to 40 inches (25 to 100 cms) is the normally used range, the adjustment capability of the device of this invention is invaluable. Moreover, when valve 106 opens to relieve the suction, the atmosphere thereby admitted into outlet 93 is prevented by valve 95 from entering collection chamber 1 and contaminating the patient. Wall suction in hospitals varies and can become hazardous if it goes up. Therefore a regulator of this type will open up more as the wall suction goes up thereby compensating for this sudden surge. It has been found so far that regulators of this type will accomodate at least half of all suction surges. A second one-way seal valve 112 of the same type as valve 95, is located in the top 113 of internal channel 105. Valve 112 comprises a resilient cap 114 outside channel 105 and exposed to the atmosphere outside the apparatus. Cap 114 has a resilient plug passing through top 113 and terminating inside channel 105 in an enlarged head 115 to retain the valve 112. Cap 114 covers perforations 116 through the top 113 of internal channel 105 and seals them against back flow of the atmosphere into channel 105. Valve 112 serves as an over positive relief valve so that if the source of suction fails in the closed position apparatus can still function as a gravity drainage system with the collection chamber 1 placed below the patient and the patient will not be jeopardized. Under these circumstances, excess pressure in the collection chamber 1 can be vented to atmosphere through valves 95 and 112, but these are one-way valves and will prevent air from flowing in the opposite direction and contaminating the patient.

An alternative form of outlet 93 is shown in Figure 11 and which provides accurate measurement of air leaks from the patient. This inlet is of the same construction as that shown in Figure 10 except that a ball flow meter 117 is located between outlet housing 94 and the top 4 of collection chamber 1. Thus, in effect, internal cavity 99 in Figure 10 has been stretched to form a ball flow meter. Consequently, valve 95 is still through the top 4 of collection chamber 1 and at the bottom of meter 117 and housing 94 is at the top of the meter. Meter 117 is a conventional ball flow meter and comprises a clear column 118 between outlet housing 94 and the top 4 of collection chamber 1. Column 118 has an internal bore containing a ball indicator 119. The bore is open at the top to internal outlet cavity 99 (Figure 10) and at the bottom through perforations 98 (Figure 10) in the top 4 of collection chamber 1. The bore is conically shaped, being narrower at the bottom than the top. The outer surface of column 118 is marked with graduations 120. Air drawn through the collection chamber 1 from the patient passes through column 118 on its way through the outlet. This flow of air drives the ball 119 upwardly in column 118 whereupon the height of the ball can be read off on graduations 120 to obtain a measure of the air flow rate. This rate can be a valuable indicator to medical staff to inform them of the severity and extent of air leaks in the patient.

Another form of outlet 93 is shown in Figure 12 which permits more accurate control over the level of suction applied to chamber 1. This outlet comprises a first housing 121 on the top 4 of chamber 1 over one-way seal valve 95 and forming a compartment 122 above valve 95. Housing 121 is sealed against flow of air from the atmosphere into compartment 122 and comprises an inner side wall 123, an outer side wall 124, rear and front walls (not shown) and a top 125. Another one-way seal valve 126, identical in construction to valve 95, is set in the outer wall 124 of housing 121 and covers perforations 127 through that wall. Valve 126 serves the same function as valve 112 in Figure 10 and allows excess positive pressure in chamber 1 to be relieved by the opening of valve 95 to compartment 122 and valve 126 to the atmosphere outside that compartment, as described above in relation to valve 112. To protect valve 126, circular cap 128 surrounds valve 126 on the outside of wall 124. The circumference of cap 128 is sealed to wall 124 but a central aperature 129 in cap 128 allows communication of the atmosphere to valve 126. Top 125 carries a second housing 130 similarly having an inner side wall 131, an outer side wall 132, front and rear walls (not shown) and a top 133 forming another compartment 134 therein. Compartment 134 is open to the atmosphere through apertures 131a and 132a in side walls 131 and 132, respectively. A circular diaphragm 135 is set in the top 125 of first housing 121 and is exposed on either side to compartments 122 and 134. Diaphragm 135 is flexible and fluid-impervious and is preferably made of neutral or silicone rubber. A stepped retaining ring 136 surrounds diaphragm 135 which is sealed around its periphery between an internal step in ring 136 and a flange 137 projecting downwardly from an upper retaining ring 138. The peripheral areas of rings 136 and 138 are sealed together and also to housings 121 and 131 thereby completing a fluid tight seal between compartments 122 and 134. Lower ring 136 has a central aperature 139 through which projects the narrow tip 140 of an outlet pipe 141. Pipe 141 extends downwardly and then turns through elbow 142 to pass sealably through the inner wall 123 of housing 121. Outside housing 121, pipe 141 turns upwardly through another elbow 143 and terminates in a connector 144 for attachment of an outlet tubing 101 for connection to a source of suction (not shown). The upper side of diaphragm 135 has an integral, central socket 145 which is accessible through a central aperature 146 in upper retaining ring 138 by a shaft 147 terminating at its lower end in a ball-shaped projection 148 seated in socket 145. Shaft 147 has a similar ball-shaped projection 149 at its upper end below which is located a washer 150 on shaft 147 which is prevented from moving along shaft 147. Washer 150 retains a spring 151 coiled around shaft 147 and extending upwardly through compartment 134 to be held at its upper end by a similar retaining arrangement attached to the bottom of an adjustment screw 152 which extends upwardly through the top 133 of housing 131. The retaining arrangement on screw 152 comprises a shaft 153 projecting downwardly therefrom and terminating in a ball-shaped projection 154. Above projection 154, a washer 155 around shaft 153 which is prevented from moving along shaft 153 retains the upper end of spring 151. Adjustment screw 152 extends through top 133 and an internally threaded column 156 set in top 133. Screw 152 is adjustable through a nut 157 fixed to the outside and top of column 156. Spring 151 is in tension between adjustment screw 152 and diaphragm 135. When suction is applied through pipe 141 the pressure in compartment 134 decreases, valve 95 opens and suction is applied to chamber 1 to encourage drainage of fluids from the patient. Also, diaphragm 135 tends to be drawn downwardly towards the tip 140 of pipe 141 and moves against the tension set in spring 151 by adjustment screw 152 which tension tends to draw diaphragm 135 upwardly and away from the tip 140 of pipe 141. At a certain level of suction, determined by adjustment screw 152, the suction will overcome the spring force and diaphragm 135 will be drawn into contact with tip 140 to cover it and close pipe 141, thereby cutting off the source of suction to chamber 1 and limiting the level of suction that can be applied to the patient. The source of suction to chamber 1 will be shut off until the level of suction in chamber 1 falls due to fluids entering chamber 1 from the patient when the suction within the chamber 1 falls, the force of spring 151 overcomes the suction and pulls the diaphragm 135 away from the tip 140 of pipe 141 to once again expose chamber 1 to the source of suction. The area of tip 140 should be small in relation to the area of diaphragm 135 and the diaphragm should be sufficiently resilient that it does not flex unduly and become fixed to tip 140 by excess suction. This arrangement of outlet has the advantage that it operates to limit excess suction very rapidly and completely.

For connection in use, the drainage apparatus is usually supplied with inlet and outlet tubes 28 and 101, respectively, for collection chamber 1 and attached as shown in Figure 1. Inlet tube 28 is for connection to the patient and outlet tube 101 is for connection to the source of suction. To avoid any error in connecting the tubes correctly, the tube 28 may be marked with the word "patient" at intervals along its length. Similarly, the outlet tube 101 may be marked with "wall suction", or similar wording. In addition, or alternatively, the tubes may be color-coded for correlation with instructions accompanying the device. The tubes are preferably clear plastic for visibility of their contents, but they may be latex or other suitable flexible synthetic material. Near their connection points to the device, each tube may be provided internally or externally with a length of coil spring around the tube to prevent the tubes from kinking.

The drainage apparatus incorporates a pressure gauge to measure directly the pressure in collection chamber 1 and provides valuable information to the hospital staff on what level of suction is actually being applied to the patient. As shown in Figures 1 and 2, pressure gauge 158 is located on the top 4 of the collection chamber 1 between the inlet 11 and outlet 93. As shown in Figure 9, the gauge 158 has a base plate 159 having a central hole 160. Adjacent hole 160 and between that hole and the front 5 of collection chamber 1, there is a wall 161 perpendicular to base plate 159 and parallel to front face 5. A transparent window 162 slopes downwardly between the top of wall 161 and the front edge of base plate 159. Triangular side walls 163 (Figures 2 and 9) which may be clear or opaque form, together with wall 161, window 162 and base 159, a housing for displaying the measured pressure on a graduated scale 164 marked in an arc on the front face of wall 161, as can best be seen in Figure 1. A shaft 165, rotatably mounted in a hole through wall 161 at the centerpoint of the arc of scale 164, carries a pointer 166 for indicating the reading of gauge 158 on scale 164. The other end of shaft 165 is rotatably mounted in a hole through an upright 167 from base 159 on the other side of central hole 160 from wall 161. Upright 167 is supported by a strut 168 between its top and wall 161. Shaft 165 carries a pinion gear 169 between wall 161 and upright 167, meshed with a vertical rack gear 170 (as can be seen in Figure 9). The rack gear 170 has helical gear teeth, in the form of a screw, to permit calibration of gauge 158 as described below. The bottom of the rack gear 170 projects through central hole 160 in base plate 159 and has a ball-shaped projection 171 rotatably secured in an integral socket 172 at the center of a circular diaphragm 173. Diaphragm 173 is flexible and fluid-impervious and is preferably made of natural or silicone rubber. A stepped retaining ring 174 surrounds diaphragm 173 which is sealed around its periphery in an internal step in ring 174 and between that step a downward, circular projection 175 from base 159. The bottom of ring 174 has a central hole 176 for communication between the interior of chamber 1 and diaphragm 175. The outer wall 177 of retaining ring 174 is sealed in a hole (not shown) in the top 4 of collection chamber 1 and an outwardly projecting flange 178 from the top of wall 177 overlaps the outer surface of top 4 of the collection chamber 1 to better retain and seal the assembly in the hole in top 4. Bottom wall 179 also prevents diaphragm 173 from bulging excessively in the case of "momentary high suction condition from the patient. The underside of base plate 159 is sealed to the upper surface of flange 178 and, since diaphragm 1763 is impervious, the gauge 158 is therefore sealably set into the top 4 of collection chamber 1 to exclude the atmosphere from the chamber.

The upper end of rack gear 170 is rotatably held in a hole in strut 167. The top of the rack gear 170 has an integral screw fitting 180 for calibrating the gauge 158 by rotating the rack gear 170 in the ball and socket joint 171/172 at its base. Such rotation operates the rack gear 170 as a worm gear, so that the position of the pinion 169 may be adjusted for a given position of the diaphragm 173. However, there is sufficient friction between the flexible diaphragm and the gear to maintain the gear position once set. Thus, with the chamber 1 at atmospheric pressure, the screw fitting 135 may be rotated to adjust the pointer 166 to zero. We have found that a small amount of stretch in the diaphragm 173 tends to result in the pointer 166 returning to zero, since there is always a small percentage of permanent set in the rubber of the diaphragm 173 when stretched up and down. The residual stretch overcomes this and brings the gauge 153 back to zero and increases the accuracy over the remainder of the scale 164 since the position is relative. Preferably, diaphragm 173 is sealed in retaining ring 174 with some radial stretch. In use, the pressure in the chamber acts on the diaphragm, relatively positive pressure bulging it upwards, relatively negative pressure bulging it downwards. The rack gear 170 moves with the diaphragm and this motion is translated through the pinion 169 to the pointer 166 to indicate accurately the pressure in the chamber 1 on the scale 164. The scale 164 may be graduated in any appropriate range of units. Conveniently, the gauge 158 may be marked to read directly in cm of water suction, for example from 0 to 60 cm in 2 cm suction intervals, since these units of measurement are commonly used in connection with hospital vacuum sources. Also graduations can be provided to indicate 1 and 2 cm pressure from the patient when the apparatus is used in gravity drainage, as previously explained.

The drainage apparatus is also provided with features which enable the hospital staff to handle the device more easily and safely. The top 4 of the collection chamber 1 is fitted with hangers 181 and 182, shown in Figure 1, for suspending the device, for example from a bed. Each hanger 181 and 182 consists of an elongate portion 183 terminating in an open hook 184 for suspending the collection chamber. The other end of each hanger terminates in a closed hook 185 engaging an eyelet 186 through a hole 187 therein. Each eyelet 186 is located towards the rear of the top 4 of the chamber 1, and projects upwardly therefrom, one behind the inlet 11 and the other behind the outlet 93, as can be seen more clearly in Figure 2. Hangers 181 and 182 are made of a material such as polypropylene strong enough to support collection chamber 1 when full of drained liquid and yet flexible enough to allow the top of each hanger to come together so that their open hooks 184 engage each other to interlock and form a carrying handle for the device, as indicated in phantom in Figure 1. Also, the hangers 181 and 182 can fold downwards onto the top 4 of chamber 1 for storage by nesting one on top of the other. Clearly, the length of the elongate portions 183 of the hangers must be such as to allow this formation of a handle and the nesting of the hangers.

The base 3 of the collection chamber is provided with a recess 189 extending across the depth of the bottom 3 of the chamber 1 and along a major portion of its length. The recess 189 accommodates, in storage, a foot 190 rotatably mounted at its center point to the bottom 3 of the collection chamber. When the chamber is to be placed on a flat surface, such as the floor or a gurney, the foot 190 can be rotated out, as indicated shown in Figure 1, to a position substantially perpendicular to the front and rear walls of the chamber, to stabilize the device against upset. During shipment, or when the device is hung from hangers 180, 181, the foot 190 may be rotated into alignment with the bottom 3 of the chamber 1, fitting within the recess 189 without protruding therefrom. To retain foot 190 in recess 189, a hole 191 (Figure 1), on the longitudinal axis of foot 190 and on either side of its central point, engage detents (not shown) located on the base 3 of chamber 1.

## Claims

1. An apparatus for draining fluid from a cavity in the body of a patient, said apparatus comprising:

   a chamber (1) for receiving said fluid;

   an inlet (11) to said chamber (1) for connection to said cavity; and

   an outlet (93) from said chamber (1) for connection to a source of vacuum, characterized in that said outlet comprises means (102) in said outlet (93) for venting said outlet (93) to the atmosphere at a predetermined level of suction and valve means (95) for allowing air to be drawn through the outlet (93) from said chamber but preventing air from entering said chamber (1) when said venting means (102) vents to the atmosphere.

2. An apparatus as claimed in Claim 1, wherein said venting means (102) comprises a seal (106) over an opening (104) in said outlet (93) to the atmosphere, a spring (107) biasing said seal (106) to close said opening (104), and a screw (110) which is adjustable to vary the compression of said spring (107) and thereby the biasing force of said spring (107) on said seal (106) and consequently to set the degree of suction required to overcome said force to open said seal (106).

3. An apparatus as claimed in Claim 1 or 2 , wherein said outlet (93) additionally comprises a valve (112) between the atmosphere outside said outlet (93) and the interior of said outlet (93) for relieving excess positive pressure in said interior of said outlet (93).

4. An apparatus as claimed in any of Claims 1 to 3 , characterized in that said apparatus includes an aperture in fluid communication with the interior of said chamber (1) and said apparatus further comprises a gauge (158), said gauge (158) comprising:

   a flexible membrane (173) positioned across said aperture and sealed to the perimeter of said aperture, said membrane (173) being impervious to said fluid and air, and providing a seal for said aperture, said membrane (173) deflecting in response to pressure differentials between the interior of said chamber (1) and the atmosphere; and

   an indicator (166) connected to said membrane (173) to move in response to deflections of said membrane (173) for providing a measurement of said pressure differentials.

5. An apparatus as claimed in claim 4, wherein said membrane (173) is stretched across said aperture.

6. An apparatus as claimed in Claim 4 or 5, further comprising a follower (170) connected to said membrane (173) for movement in response to deflection of said membrane, said indicator (166) being responsive to said movement of said follower (170) for providing said pressure differential measurement.

7. An apparatus as claimed in Claim 6, wherein said follower (170) comprises a threaded shaft (170) rotatably connected to said membrane (173) and mounted on said membrane (173) for reciprocal movement therewith, and wherein said indicator includes a gear (169) engaging with, and rotatable by, said shaft (170).

8. An apparatus as claimed in Claim 7, wherein said shaft (170) terminates at its end opposite said membrane (173) in a fitting (100) for receiving a tool for rotating said shaft (170) and thereby calibrating said gauge (158).

9. An apparatus as claimed in any preceding claim, characterized in that said inlet (11) includes an inlet passage having:

   means (24, 25) for conducting said fluid from said body cavity upwardly before said fluid is received in said chamber (1); and

   means (19, 20, 21) for selectively removing said upward conducting means from the path of said fluid in said inlet passage.

10. An apparatus as claimed in Claim 9 , wherein said inlet passage comprises a first wall depending downwardly from the top of said passage, wherein said upwardly conducting means comprises a second wall (25) extending upwardly from the bottom of said passage, said second wall (25) terminating below the top (14) of said passage at a height above the bottom of said first wall, and wherein said selectively removing means comprises means for selectively causing fluid entering said inlet passage to either (a) flow beneath said first wall and over the second wall, or (b) flow into said chamber without flowing over said second wall.

11. An apparatus as claimed in Claim 9 or 10 , wherein said inlet passage is movable with respect to said chamber (1).

12. An apparatus as claimed in Claim 11, wherein said inlet passage is located in an inlet compartment (12), said second wall (25) extending between opposite sides of said compartment to form within said compartment said inlet passage and an overflow passage.

13. An apparatus as claimed in Claim 12, wherein said compartment (12) is movable from said one position, in which the bottom of said inlet passage is sealed and the bottom of said overflow passage is open to said chamber (1) so that drained fluid passes upwardly over said second wall (25) and through said overflow passage to said chamber (1); to another position, in which the bottom of said overflow passage is sealed and the bottom of said inlet passage is open to said chamber (1) so that drained fluid passes through the bottom of said inlet passage into said chamber (1) without passing upwardly.

14. An apparatus as claimed in Claim 13 , wherein said inlet compartment (12) is movable from said one position to said another position by rotation.

15. An apparatus as claimed in Claim 14, wherein said inlet compartment (12) comprises an outer perimeter wall (13) extending upwardly from the top of said chamber (1), said wall having an outwardly projecting, circular flange (19) at its bottom and wherein said chamber (1) includes an aperture (30) through the top of said chamber (1), a retaining ring (20) attached to said top and around said aperture, said ring (20) having an outwardly projecting flange (21) surrounding and overhanging said inlet compartment flange (19) to retain said compartment on the top of said chamber, said compartment (12) being movable by rotation within said retaining ring (20) to place either said inlet passage or said overflow passage over said aperture through the top of said chamber.

16. An apparatus as claimed in Claim 12, wherein said inlet compartment (69) is movable from said one position to said another position by sliding.

17. An apparatus as claimed in Claim 16, wherein said inlet compartment comprises an outer perimeter wall (70, 71, 72, 73) extending upwardly from the top of said chamber (1), said wall having outwardly extending, parallel flanges (75, 76) at the bottom of at least two opposite sides (70, 71) of said outer wall, said chamber including an aperture (92) through the top of said chamber and parallel guide rails (77) attached to said top, said flange, overhanging one of said compartment flanges to retain said compartment on the top of said chamber, so that said compartment is movable by sliding along said rails (77), said apparatus further comprising stops (81, 82) between said rails which limit the extent of travel of said compartment so that either said inlet passage or said overflow passage is placed over said aperture.

18. An apparatus as claimed in any one of Claims 11 to 17, wherein said inlet compartment (12) is movable without cutting off the supply of drained fluid into said chamber (1).

19. An apparatus as claimed in Claim 10, wherein said first and second walls are portions of a continuous wall which extends from the bottom of said first wall and the top of said second wall, and wherein said inlet passage further comprises a pair of side walls sealed to opposite edges of said continuous wall, said inlet passage also including an inlet aperture in one of said side walls, said selectively remaining means comprising means for moving said inlet passage by rotation to selectively position the top of said second wall below the bottom of the first wall to spill the contents of said inlet passage into said chamber.

20. An apparatus as claimed in Claim 10, wherein said chamber has a first inlet, positioned to supply said fluid to said inlet passage, and a second inlet, positioned to cause said fluid to bypass said inlet passage and flow directly into said chamber, said selectively removing means comprising means for switching the flow of drained fluid from said first inlet to said second inlet, and vice versa.

21. An apparatus as claimed in Claims 19 or 20, wherein said selectively removing means operates without cutting off the supply of drained fluid into said chamber.

22. An apparatus as claimed in any preceding claim, additionally comprising sampling access means to permit withdrawal of drained fluid from said inlet passage.

23. An apparatus as claimed in any preceding claim, characterized by further comprising: means (181) for suspending said chamber (1); and means (190) for stabilizing said chamber (1) on a support surface, said stabilizing means comprising a stand (190) rotatably mounted on the base (3) of said chamber between a first position in which said stand lies within the boundary of said base and a second position in which said stand extends beyond the boundary of, and thereby stabilizes, said base.

24. An apparatus as claimed in Claim 23, wherein said suspending means (181) are foldable against said chamber (1) for storage and extendable to form a carrying handle for said chamber and wherein said stand (190) is rotatably and centrally mounted in a recess (189) in said base of said chamber, said stand being sized to be stored completely within said recess.

25. An apparatus as claimed in Claim 24, wherein said suspending means (181) comprises a pair of hangers (181, 182), each comprising a central, elongate portion (183) terminating at one end in an open hook (184) for suspending said chamber (1) and terminating at the other end in a closed hook (184), and wherein said chamber comprises a pair of eyelets (186) attached to said closed hooks (184), said hangers (181, 182) being sized so that they can be alternatively (a) nested upon each other between said eyelets or (b) engage each other by means of the open hooks to form a carrying handle.

26. An apparatus as claimed in any of Claims 1 to 8, wherein said inlet comprises a generally U-shaped inlet passage, through which said fluid will pass before spilling into said chamber (1), said passage being for holding a reservoir of liquid in the bottom of said passage; and means for dumping said reservoir of liquid into said chamber (1).

27. An apparatus as claimed in any of Claims 1 to 8, further comprising means for intermittently placing in the path of said fluid into said chamber said generally U-shaped inlet passage so that, when said passage is placed into said path, said fluid passes through a reservoir of liquid at the bottom of said passage before spilling out of said passage into said chamber (1), said passage in said path causing an air leak from said cavity to be displayed as a bubbling of air through said liquid reservoir.

28. An apparatus as claimed in Claim 26, further comprising means for sampling from said reservoir liquid most recently drained from said cavity.

29. An apparatus as claimed in any preceding Claim comprising: means for creating a substantially horizontal gas/liquid interface in said inlet in the path of said fluid into said chamber; and means for removing said interface from said path of said fluid.

**Patentansprüche**

1. Vorrichtung zum Ablassen von Fluid aus einem Hohlraum in dem Körper eines Patienten, wobei die Vorrichtung folgende Merkmale aufweist:
   - Eine Kammer (1) zur Aufnahme des Fluids;
   - einen Einlaß (11) zu der Kammer (1) zur Verbindung mit dem Hohlraum; und
   - einen Auslaß (93) von der Kammer (1) zur Verbindung mit einer Vakuumquelle, dadurch **gekennzeichnet**, daß der Auslaß eine Einrichtung (102) in dem Auslaß (93) zur Entlüftung des Auslasses (93) zur Umgebung bei einem vorbestimmten Absaugungsniveau aufweist, sowie Ventileinrichtungen (95), die ermöglichen, daß Luft durch den Auslaß (93) von der Kammer gezogen wird, aber die verhindern, daß Luft in die Kammer (1) eintritt, wenn die Entlüftungseinrichtung (102) zur Umgebung hin entlüftet.

2. Vorrichtung nach Anspruch 1, bei der die Entlüftungseinrichtung (102) eine Dichtung (106) über einer Öffnung (104) in dem Auslaß (93) zur Umgebung aufweist, eine Feder (107), die die Dichtung (106) vorspannt, um die Öffnung (104) zu schließen, sowie eine Schraube (110), die einstellbar ist, um die Kompression der Feder (107) und dadurch die Vorspannungskraft der Feder (107) auf die Dichtung (106) zu verändern, und um folglich den Absaugungsgrad einzustellen, der benötigt wird, um die Kraft zur Öffnung der Dichtung (106) zu überwinden.

3. Vorrichtung nach Anspruch 1 oder 2, bei der der Auslaß (93) zusätzlich ein Ventil (112) zwischen der Umgebung außerhalb des Auslasses (93) und dem Inneren des Auslasses (93) aufweist, um überschüssigen positiven Druck in dem Inneren des Auslasses (93) zu entspannen.

4. Vorrichtung nach einem der Ansprüche 1 bis 3, dadurch **gekennzeichnet**, daß die Vorrichtung eine Öffnung aufweist, die in Fluidverbindung mit dem Inneren der Kammer (1) steht, und daß die Vorrichtung weiterhin ein Meßinstrument (158) aufweist, das folgende Merkmale hat:
   - Eine flexible Membran (173), die quer über der Öffnung angeordnet ist und abdichtend am Umfang der Öffnung angebracht ist, wobei die Membran (173) undurchlässig für das Fluid und für Luft ist und eine Dichtung für die Öffnung bildet, wobei sich die Membran (173) in Reaktion auf Druckunterschiede zwischen dem Inneren der Kammer (1) und der Umgebung wölbt; und
   - einen Anzeiger (166), der mit der Membran (173) verbunden ist, um sich in Reaktion auf die Wölbungen der Membran (173) zu bewegen, um eine Messung der Druckunterschiede zu liefern.

5. Vorrichtung nach Anspruch 4, bei der die Membran (173) quer über die Öffnung gespannt ist.

6. Vorrichtung nach Anspruch 4 oder 5, die weiterhin ein Folgeglied (170) aufweist, das mit der Membran (173) zur Bewegung in Reaktion auf die Wölbung der Membran verbunden ist, wobei der Anzeiger (166) auf die Bewegung des Folgeglieds (170) reagiert, um die Messung des Druckunterschieds zu liefern.

7. Vorrichtung nach Anspruch 6, bei der das Folgeglied (170) einen mit einem Gewinde versehenen Schaft (170) aufweist, der drehbar mit der Membran (173) verbunden ist und an der Membran (173) für eine hin- und hergehende Bewegung mit dieser angebracht ist, und wobei dar Anzeiger eine Übersetzung (169) aufweist, die in den Schaft (170) eingreift und durch diesen drehbar ist.

8. Vorrichtung nach Anspruch 7, bei der der Schaft (170) an seinem der Membran (173) gegenüberliegenden Ende in einer Armatur (180) endet, in der ein Werkzeug zur Drehung des Schaftes (170) aufgenommen ist und wodurch das Meßinstrument (158) kalibriert wird.

9. Vorrichtung nach einem der vorangehenden Ansprüche, dadurch **gekennzeichnet**, daß der Einlaß (11) einen Einlaßdurchgang aufweist, der folgende Merkmale aufweist:
   - Eine Einrichtung (24, 25), die das Fluid von dem Körperhohlraum nach oben leitet, bevor das Fluid in der Kammer (1) aufgenommen wird; und
   - eine Einrichtung (19, 20, 21) zur wahlweisen Entfernung der aufwärts leitenden Einrichtung von der Bahn des Fluids in dem Einlaßdurchgang.

10. Vorrichtung nach Anspruch 9, bei der der Einlaßdurchgang eine erste Wand aufweist, die sich von dem Oberteil des Durchganges nach unten erstreckt, bei der die aufwärts leitende Einrichtung eine zweite Wand (25) aufweist, die sich von dem Unterteil des Durchganges nach oben erstreckt, wobei die zweite Wand (25) unter dem Oberteil (14) des Durchganges in einer Höhe über dem Unterteil der ersten Wand endet, und wobei die Einrichtung zur wahlweisen Entfernung Mittel aufweist, die wahlweise bewirken, daß Fluid, das in den Einlaßdurchgang eintritt, entweder (a) unterhalb der ersten Wand und über die zweite Wand fließt, oder (b) in die Kammer fließt, ohne über die zweite Wand zu fließen.

11. Vorrichtung nach Anspruch 9 oder 10, bei der der Einlaßdurchgang in Bezug auf die Kammer (1) bewegbar ist.

12. Vorrichtung nach Anspruch 11, bei der der Einlaßdurchgang in einem Einlaßraum (12) angeordnet ist, wobei sich die zweite Wand (25) zwischen gegenüberliegenden Seiten des Raumes erstreckt, um innerhalb des Raumes den Einlaßdurchgang und einen Überlaufdurchgang zu bilden.

13. Vorrichtung nach Anspruch 12, bei der der Raum (12) von der einen Position, bei der das Unterteil des Einlaßdurchganges abgedichtet und das Unterteil des Überlaufdurchganges zu der Kammer (1) hin offen ist, so daß abgelassenes Fluid nach oben über die zweite Wand (25) und durch den Überlaufdurchgang zu der Kammer (1) durchtritt, in eine andere Position bewegbar ist, in der das Unterteil des Überlaufdurchganges abgedichtet und das Unterteil des Einlaßdurchganges zu der Kammer (1) hin offen ist, so daß abgelassenes Fluid durch das Unterteil des Einlaßdurchganges in die Kammer (1) durchtritt, ohne nach oben hin durchzutreten.

14. Vorrichtung nach Anspruch 13, bei der der Einlaßraum (12) von der einen Position in die andere Position durch Drehung bewegbar ist.

15. Vorrichtung nach Anspruch 14, bei der der Einlaßraum (12) eine äußere Umfangswand (13) aufweist, die sich von dem Oberteil der Kammer (1) nach oben erstreckt, wobei die Wand einen nach außen gerichteten vorstehenden kreisförmigen Rand (19) an ihrem Unterteil hat, und wobei die Kammer (1) eine Öffnung (30) durch das Oberteil der Kammer (1) aufweist, wobei ein Rückhaltering (20) an dem Oberteil und um die Öffnung herum angebracht ist, wobei der Ring (20) einen nach außen gerichteten vorstehenden Rand (21) hat, der den Rand (19) des Einlaßraumes umgibt und über diesen vorspringt, um den Raum auf dem Oberteil der Kammer zu halten, wobei der Raum (12) durch Drehung innerhalb des Rückhalteringes (20) bewegbar ist, um entweder den Einlaßdurchgang oder den Überlaufdurchgang über der Öffnung durch das Oberteil der Kammer anzuordnen.

16. Vorrichtung nach Anspruch 12, bei der der Einlaßraum (69) von der einen Position in die andere Position durch Verschieben bewegbar ist.

17. Vorrichtung nach Anspruch 16, bei der der Einlaßraum eine äußere Umfangswand (70, 71, 72, 73) aufweist, die sich von dem Oberteil der Kammer (1) nach oben erstreckt, wobei die Wand sich nach außen erstreckende parallele Ränder (75, 76) an dem Unterteil von mindestens zwei gegenüberliegenden Seiten (70, 71) der äußeren Wand hat, wobei die Kammer eine Öffnung (92) durch das Oberteil der Kammer sowie parallele Führungsschienen (77) aufweist, die an dem Oberteil angebracht sind, wobei der Rand über einen der Ränder des Raumes vorspringt, um den Raum auf dem Oberteil der Kammer zu halten, so daß der Raum durch Verschiebung entlang der Schienen (77) bewegbar ist, wobei die Vorrichtung weiterhin Anschläge (81, 82) zwischen den Schienen aufweist, die das Ausmaß der Bewegung des Raumes begrenzen, so daß entweder der Einlaßdurchgang oder der Überlaufdurchgang über der Öffnung angeordnet ist.

18. Vorrichtung nach einem der Ansprüche 11 bis 17, bei der der Einlaßraum (12) bewegbar ist, ohne die Zufuhr von abgelassenem Fluid in die Kammer (1) abzuschneiden.

19. Vorrichtung nach Anspruch 10, bei der die erste und die zweite Wand Abschnitte einer durchgehenden Wand sind, die sich von dem Unterteil der ersten Wand und dem Oberteil der zweiten Wand erstreckt, und wobei der Einlaßdurchgang weiterhin ein Paar Seitenwände aufweist, die abdichtend an gegenüberliegenden Kanten der durchgehenden Wand angebracht sind, wobei der Einlaßdurchgang außerdem eine Einlaßöffnung in einer der Seitenwände aufweist, wobei die Einrichtungen zur wahlweisen Entfernung Mittel aufweisen, die den Einlaßdurchgang durch Drehung bewegen, um das Oberteil der zweiten Wand wahlweise unter dem Unterteil der ersten Wand anzuordnen, um den Inhalt des Einlaßdurchgangs in die Kammer zu gießen.

20. Vorrichtung nach Anspruch 10, bei der die Kammer einen ersten Einlaß hat, der derart angeordnet ist, daß das Fluid dem Einlaßdurchgang zugeführt wird, und einen zweiten Einlaß, der derart angeordnet ist, daß bewirkt wird, daß das Fluid den Einlaßdurchgang umgeht und direkt in die Kammer fließt, wobei die Einrichtungen zur wahlweisen Entfernung Mittel aufweisen, die den Fluß des abgelassenen Fluids von dem ersten Einlaß zu dem zweiten Einlaß umlenken, und umgekehrt.

21. Vorrichtung nach Anspruch 19 oder 20, bei der die Einrichtung zur wahlweisen Entfernung arbeitet, ohne die Zufuhr von abgelassenem Fluid in die Kammer abzuschneiden.

22. Vorrichtung nach einem der vorangehenden Ansprüche, die zusätzlich eine Einrichtung zur Probenentnahme aufweist, um die Entnahme von abgelassenem Fluid von dem Einlaßdurchgang zuzulassen.

23. Vorrichtung nach einem der vorangehenden Ansprüche, dadurch **gekennzeichnet**, daß sie weiterhin folgende Merkmale aufweist:
   - Eine Einrichtung (181) zur Aufhängung der Kammer (1);
   - eine Einrichtung (190) zur Stabilisierung der Kammer (1) auf einer Abstützfläche, wobei die Stabilisierungseinrichtung eine Stütze (190) aufweist, die an der Grundfläche (3) der Kammer angebracht ist und zwischen einer ersten Position, in der die Stütze innerhalb der Grenzlinien der Grundfläche liegt, und einer zweiten Position drehbar ist, in der die Stütze sich über die Grenzlinien der Grundfläche hinaus erstreckt und diese dadurch stabilisiert.

24. Vorrichtung nach Anspruch 23, bei der die Aufhängeeinrichtung (181) zum Lagern gegen die Kammer (1) eingeklappt und zur Bildung eines Tragegriffes für die Kammer ausgeklappt werden kann und wobei die Stütze (190) drehbar und zentral in einer Ausnehmung (189) in der Grundfläche der Kammer angebracht ist, wobei die Stütze so dimensioniert ist, daß sie vollständig innerhalb der Ausnehmung untergebracht werden kann.

25. Vorrichtung nach Anspruch 24, bei der die Aufhängeeinrichtung (181) ein Paar Hängelager (181, 182) aufweist, die jeweils einen mittleren langgestreckten Abschnitt (183) aufweisen, der an einem Ende in einem offenen Haken (184) zur Aufhängung der Kammer (1) und an dem anderen Ende in einem geschlossenen Haken (185) endet, und wobei die Kammer ein Paar Ösen (186) aufweist, die an den geschlossenen Haken (185) angebracht sind, wobei die Hängelager (181, 182) so dimensioniert sind, daß sie alternativ (a) aufeinander zwischen den Ösen eingepaßt werden können oder daß sie (b) mittels der offenen Haken ineinander eingreifen, um einen Tragegriff zu bilden.

26. Vorrichtung nach einem der Ansprüche 1 bis 8, bei der der Einlaß einen im wesentlichen U-förmigen Einlaßdurchgang aufweist, durch den das Fluid hindurchtritt, bevor es in die Kammer (1) gegossen wird, wobei der Durchgang dafür vorgesehen ist, ein Flüssigkeitsreservoir in dem Unterteil des Durchganges zu halten, und wobei Einrichtungen vorgesehen sind, die das Flüssigkeitsreservoir in die Kammer (1) entleeren.

27. Vorrichtung nach einem der Ansprüche 1 bis 8, die weiterhin Einrichtungen aufweist, um den im wesentlichen U-förmigen Einlaßdurchgang zeitweise in der Bahn des Fluids in die Kammer so anzuordnen, daß, wenn der Durchgang in der Bahn angeordnet ist, das Fluid durch ein Flüssigkeitsreservoir im Unterteil des Durchgangs durchtritt, bevor es aus dem Durchgang hinaus in die Kammer (1) hineingegossen wird, wobei der Durchgang in der Bahn bewirkt, daß ein Luftleck in dem Hohlraum in Form von Luftblasen in dem Flüssigkeitsreservoir dargestellt wird.

28. Vorrichtung nach Anspruch 26, die weiterhin Einrichtungen aufweist, durch die aus der Flüssigkeit, die unmittelbar vorher von dem Hohlraum abgelassen worden ist, Proben aus dem Reservoir entnommen werden können.

29. Vorrichtung nach einem der vorangehenden Ansprüche, die folgende Merkmale aufweist:
   - Einrichtungen zur Erzeugung einer im wesentlichen horizontalen Gas/Flüssigkeits-Grenzfläche in dem Einlaß in der Bahn des Fluids in die Kammer; und
   - Einrichtungen zur Entfernung der Grenzfläche aus der Bahn des Fluids.

**Revendications**

1. Appareil de drainage pour évacuer un fluide d'une cavité du corps d'un patient, l'appareil comprenant :
   une chambre (1) pour recevoir le fluide;
   une entrée (11) de ladite chambre (1), agencée pour être raccordée à ladite cavité; et
   une sortie (93) de ladite chambre (), agencée pour être raccordée à une source de vide, caractérisé en ce que ladite sortie comporte des moyens de mise à l'air (102) disposés dans ladite sortie (93) pour la mettre en communication avec l'atmosphère à un niveau prédéterminé d'aspiration et des moyens à soupape (95) permettant que de l'air soit tiré de ladite chambre à travers la sortie (93), mais empêchant que de l'air entre dans ladite chambre (1) quand les moyens de mise à l'air (102) réalisent la communication avec l'atmosphère.

2. Appareil selon la revendication 1, dans lequel les moyens de mise à l'air (102) comportent un obturateur (106) sur un orifice (104) reliant ladite sortie (93) à l'atmosphère, un ressort (107) sollicitant l'obturateur (106) pour fermer ledit orifice (104), et une vis (110) qui est réglable pour faire varier la compression du ressort (107) et donc la force de poussée du ressort (107) sur l'obturateur (106) et régler ainsi le degré d'aspiration nécessaire pour vaincre ladite force pour ouvrir l'obturateur (106).

3. Appareil selon la revendication 1 ou 2, dans lequel ladite sortie (93) comporte en outre une soupape (112) entre l'atmosphère entourant la sortie (93) et l'intérieur de la sortie (93) pour libérer une pression positive excessive à l'intérieur de cette sortie (93).

4. Appareil selon l'une des revendications 1 à 3, caractérisé en ce qu'il comporte une ouverture communiquant avec l'intérieur de ladite chambre (1) et en ce que l'appareil comporte en outre une jauge (158), ladite jauge (158) comprenant :

une membrane flexible (173) disposée à travers ladite ouverture et jointe de manière étanche au périmètre de l'ouverture, cette membrane (173) étant imperméable audit fluide et à l'air et assurant une fermeture étanche de ladite ouverture, ladite membrane (173) fléchissant en réponse à une différence de pression entre l'intérieur de la chambre (1) et l'atmosphère; et

un indicateur (166) raccordé à la membrane (173) de façon à se déplacer en réponse à des déflexions de la membrane (173) pour fournir une mesure de ladite différence de pression.

5. Appareil selon la revendication 4, dans lequel la membrane (173) est tendue à travers ladite ouverture.

6. Appareil selon la revendication 4 ou 5, comportant en outre un élément suiveur (170) raccordé à la membrane (173) de façon à se déplacer en réponse à une déflexion de la membrane, ledit indicateur (166) étant sensible au déplacement de l'élément suiveur (170) pour fournir ladite mesure de différence de pression.

7. Appareil selon la revendication 6, dans lequel l'élément suiveur (170) comporte une tige filetée (170) raccordée de manière rotative à la membrane (173) et montée sur la membrane (173) de façon à se déplacer en va-et-vient avec elle, et dans lequel l'indicateur comporte une roue dentée (169) engrenée sur ladite tige (170) et entraînée en rotation par elle.

8. Appareil selon la revendication 7, dans lequel ladite tige (170) se termine, à son extrémité opposée à la membrane (173), par un embout (180) destiné à recevoir un outil destiné à faire tourner la tige (170) et calibrer ainsi la jauge (158).

9. Appareil selon l'une des revendications précédentes, caractérisé en ce que ladite entrée (11) comprend un passage d'entrée comportant :

des moyens (24, 25) conduisant vers le haut le fluide issu de la cavité du corps avant que le fluide soit recueilli dans ladite chambre (1); et

des moyens (19, 20, 21) pour rétracter sélectivement lesdits moyens conduisant vers le haut, en les ôtant du parcours du fluide dans ledit passage d'entrée.

10. Appareil selon la revendication 9, dans lequel ledit passage d'entrée comporte une première paroi descendant du haut dudit passage, dans lequel lesdits moyens conduisant vers le haut comportent une seconde paroi (25) s'étendant vers le haut à partir du bas dudit passage, cette seconde paroi (25) se terminant en dessous du sommet (14) du passage à un niveau supérieur à celui du bas de la première paroi, et dans lequel lesdits moyens pour rétracter sélectivement comportent des moyens pour diriger sélectivement le fluide entrant dans le passage d'entrée afin que le fluide (a) s'écoule par-dessous la première paroi et par-dessus la seconde paroi, ou (b) s'écoule vers ladite chambre sans passer par-dessus la seconde paroi.

11. Appareil selon la revendication 9 ou 10, dans lequel ledit passage d'entrée est mobile par rapport à ladite chambre (1).

12. Appareil selon la revendication 11, dans lequel ledit passage d'entrée est situé dans un compartiment d'entrée (12), ladite seconde paroi (25) s'étendant entre des côtés opposés du compartiment pour former ledit passage d'entrée et un passage de trop-plein à l'intérieur du compartiment.

13. Appareil selon la revendication 12, dans lequel ledit compartiment (12) est mobile à partir de la première position, dans laquelle le fond du passage d'entrée est obturé et le fond du passage de trop-plein est ouvert en direction de ladite chambre (1), de sorte que le fluide drainé passe par le haut au-dessus la seconde paroi (25) et à travers le passage de trop-plein jusqu'à ladite chambre (1), jusqu'à une autre position dans laquelle le fond du passage de tropplein est obturé et le fond du passage d'entrée est ouvert en direction de ladite chambre (1), de sorte que le fluide drainé passe à travers le fond du passage d'entrée en direction de ladite chambre (1) sans passer par le haut.

14. Appareil selon la revendication 13, dans lequel le compartiment d'entrée (12) est mobile de la première position à l'autre position par rotation.

15. Appareil selon la revendication 14, dans lequel le compartiment d'entrée (12) comporte une paroi périphérique extérieure (13) s'étendant vers le haut à partir du sommet de ladite chambre (1) et ayant un rebord circulaire (19) dirigé vers l'extérieur au bas de la paroi, et dans lequel ladite chambre (1) comporte une ouverture (30) à travers le sommet de la chambre (1), un anneau de retenue (20) solidaire du sommet de la chambre et entourant ladite ouverture, ledit anneau (20) comportant un rebord (21) dirigé vers l'extérieur de façon à entourer et recouvrir ledit rebord (19) du compartiment d'entrée pour retenir ce compartiment sur le sommet de la chambre, le compartiment (12) étant mobile par rotation à l'intérieur de l'anneau de retenue (20) de façon à placer soit le passage d'entrée, soit le passage de trop-plein sur l'ouverture du sommet de ladite chambre.

16. Appareil selon la revendication 12, dans lequel le compartiment d'entrée (69) est mobile de la première position à l'autre position par coulissement.

17. Appareil selon la revendication 16, dans lequel le compartiment d'entrée comporte une paroi périphérique extérieure (70, 71, 72, 73) s'étendant vers le haut à partir du sommet de ladite chambre (1), cette paroi ayant des rebords parallèles (75, 76) s'étendant vers l'extérieur au bas d'au moins deux côtés opposés (70, 71) de la paroi extérieure, ladite chambre comportant une ouverture (92) à travers le sommet de la chambre et des rails parallèles de guidage (77) solidaires dudit sommet, un rebord recouvrant l'un des rebords du compartiment pour retenir le compartiment sur le sommet de ladite chambre, de sorte que le compartiment est mobile par glissement le long des rails (77), l'appareil comportant en outre des arrêts (81, 82) entre les rails pour limiter la course du compartiment de façon que soit le passage d'entrée, soit le passage de trop-plein soit placé sur ladite ouverture.

18. Appareil selon l'une des revendications 11 à 17, dans lequel le compartiment d'entrée (12) peut être déplacé sans interrompre l'arrivée du fluide drainé dans ladite chambre (1).

19. Appareil selon la revendication 10, dans lequel la première et la seconde paroi sont des parties d'une paroi continue qui s'étend à partir du bas de la première paroi et du haut de la seconde paroi, et dans lequel le passage d'entrée comporte en outre une paire de parois latérales jointes hermétiquement à des bords opposés de la paroi continue, le passage d'entrée comportant également une ouverture d'entrée dans l'une des parois latérales, lesdits moyens pour rétracter sélectivement comprenant des moyens pour déplacer le passage d'entrée par rotation afin de positionner sélectivement le sommet de la seconde paroi en dessous du bas de la première paroi pour déverser dans la chambre le contenu du passage d'entrée.

20. Appareil selon la revendication 10, dans lequel ladite chambre comporte une première entrée, placée de façon à délivrer le fluide dans ledit passage d'entrée, une seconde entrée placée de façon à court-circuiter le passage d'entrée et faire aller le fluide directement dans la chambre, lesdits moyens pour rétracter sélectivement comportant des moyens pour aiguiller l'écoulement du fluide drainé entre la première entrée et la seconde entrée et vice-versa.

21. Appareil selon la revendication 19 ou 20, dans lequel lesdits moyens pour rétracter sélectivement fonctionnent sans interrompre l'arrivée du fluide drainé dans la chambre.

22. Appareil selon l'une des revendications précédentes, comportant en outre des moyens d'accès d'échantillonnage pour permettre de prélever du fluide drainé dans le passage d'entrée.

23. Appareil selon l'une des revendications précédentes, caractérisé en ce qu'il comporte en outre :
   des moyens (181) pour suspendre ladite chambre (1); et
   des moyens (190) pour stabiliser ladite chambre (1) sur une surface de support, les moyens de stabilisation comportant un support (190) monté sur la base (3) de la chambre de manière à pouvoir tourner entre une première position dans laquelle le support se trouve dans les limites de ladite base et une seconde position dans laquelle le support s'étend au-delà des limites de ladite base de manière à la stabiliser.

24. Appareil selon la revendication 23, dans lequel lesdits moyens de suspension (181) sont rabattables contre la chambre (1) pour le stockage et déployables pour former une poignée de transport de la chambre, et dans lequel ledit support (190) est monté de manière rotative et centrée dans un évidement (189) de la base de la chambre, ce support étant dimensionné de manière à se ranger complètement à l'intérieur de l'évidement.

25. Appareil selon la revendication 24, dans lequel les moyens de suspension (181) comportent une paire de crochets de suspension (181, 182) dont chacun comprend une partie centrale allongée (183) se terminant à une extrémité en un crochet ouvert (184), destiné à la suspension de la chambre (1), et à l'autre extrémité en un crochet fermé (184), et dans lequel la chambre comporte une paire d'oeillets (186) accrochés aux crochets fermés (184), les crochets de suspension (181, 182) étant dimensionnés de façon à pouvoir être alternativement (a) rabattus l'un sur l'autre entre lesdits oeillets ou (b) accrochés l'un à l'autre par leur crochets ouverts pour former une poignée de transport.

26. Appareil selon l'une des revendications 1 à 8, dans lequel ladite entrée comporte un passage d'entrée en forme générale de U, à travers lequel le fluide passera avant de se répandre dans ladite chambre (1), ce passage étant agencé pour maintenir un réservoir de liquide au fond du passage, et des moyens pour déverser le réservoir de liquide dans la chambre (1).

27. Appareil selon l'une des revendications 1 à 8, comportant en outre des moyens pour placer de manière intermittente ledit passage d'entrée en forme générale de U sur le chemin du fluide allant dans la chambre de façon que, lorsque ledit passage est placé sur ce chemin, le fluide passe à travers un réservoir de liquide au bas dudit passage avant de s'écouler du passage à la chambre (1), la présence dudit passage sur le chemin du fluide ayant pour effet d'indiquer une fuite d'air venant de ladite cavité sous la forme de bulles d'air dans le réservoir de liquide.

28. Appareil selon la revendication 26, comportant en outre des moyens pour prélever dans le réservoir des échantillons du liquide recueilli dernièrement par drainage de ladite cavité.

29. Appareil selon l'une des revendications précédentes, comportant :

des moyens pour créer une interface gaz/liquide sensiblement horizontale dans ladite entrée, sur le chemin du fluide en direction de ladite chambre; et

des moyens pour retirer ladite interface du chemin du fluide.

Fig. 1

Fig. 2

Fig. 3

Fig. 4

Fig. 5

Fig. 6

Fig. 7

Fig. 8

Fig. 9

Fig. 10

Fig. 11

Fig. 12